# EUROPEAN PATENT APPLICATION

(11) **EP 3 716 024 A1**
(43) Date of publication of application: **30.09.2020**
(21) Application number: 19460013.6
(22) Date of filing: 28.03.2019
(51) Int. Cl.: G06F 3/0346, G06F 3/0354, G06F 3/0481, G06F 3/01, A61B 90/00, G06F 3/03, G06T 7/73

(54) **MULTI-STATE POINTER / MANIPULATOR FOR OPTICAL TRACKING SYSTEMS**

(71) Applicant: Politechnika Slaska, 44-100 Gliwice (PL)
(72) Inventor: Juszcyk, Jan, 43-190 Mikolów (PL); Pycinski, Bartlomiej, 40-583 Katowice (PL)

(57) **Abstract**

Multi-state marker /manipulator for optical systems which track the location of the object is characterized by the fact that it consists of five spheres [1], and two spheres are interchangeably covered and three are always visible; spheres are mounted on pins [4] connected by arms [3]; has at least two screening mechanism [5], mounted on pins [4] where screening mechanism [5] consist of two hemispheres [6] mounted on sleeve [7] equipped with a pull-off spring that pulls it towards the sphere [1]; the pull arms [8], which perform the opening of the hemisphere [6], which simultaneously deflect the hemisphere [6], are connected by the connector [10]. The connector has a hinge [11]. The control is carried out by an additional handle [12]. Spheres [1] are covered with reflective material and the tracking system is a camera.

## Description

The subject of the invention is a multi-state marker / manipulator for optical systems which track the location of the object.

Various optical tracking systems are available (e.g. NDI Polaris, Claron, PST). A common feature of these systems is the need to use special markers or geometric patterns, which are tracked and uniquely identified by the device. Markers may be implemented for example as a specific number of reflective points detected by the device (e.g., patent no. USD761428S1) or as a chessboard fragment (carried out by ClaroNav, patent no. WO2012068679A1). Specific marker is identified from the others visible by the tracking system just by its unique pattern (e.g. arrangement of reflective points, such as in US7993353B2, or a fragment of a flat pattern).

A common feature, which can be considered as a drawback of the above-mentioned markers, is their rigid geometry that can not be changed during tracking. Therefore the objects are not able to change their functions.

There are also markers with the possibility of changing their geometry, allowing to handle more tools (objects), such as those reserved by patent WO2006115551A1 or patent EP1518508A1, in which the change of one traced elements' position changes the geometry of the entire marker. A similar solution is reserved in US20050215888A1, however, in this case the change of the marker geometry is performed by changing the position of the whole arm, to which a tracked marker is attached.

The limitations of the solutions described above are the inability to change the geometry of the marker when it is being used, dependence on the power source and, the inability to dynamically change the state.

The main point of the invention is a multi-state marker / manipulator for tracking the position of a virtual object by an optical tracking system. The manipulator features at least five spheres, including two spheres interchangeably covered and the others visible, fixed on sticks and joined by arms. There are also at least two occluding mechanisms, each mounted on a stick. The occluding mechanism consists of two hemispheres attached to a sleeve with a pull-back spring and pull-off arms connected together having a free sleeve. The connector contains a hinge. The multi-state marker / manipulator contains a control handle. The optical tracking system is a camera.

The spheres are covered with reflective material. The arms are joined together stiffly.

An advantage of the invention is its passivity, i.e. there is no need to supply the manipulator with either external or internal energy sources. The autonomous mechanical solution does not require a power source. Another advantage is the possibility of dynamic change of state. A change of state is stands for a change of the marker's geometry.

It is possible to extend the functionality by a larger number of states, therefore the invention can be extended to a larger number of defined geometries within the same marker.

The solution does not require the tracking system to be replaced, which reduces the cost of its use. The solution allows to integrate more than one marker without the need to change the geometry, as the position of the spheres detected by the system does not change. With the solution, only one state of marker is being detected.

The invention is a universal solution because it does not require a change in the relative position of the spheres, thus the changeover does not affect the accuracy of the binary marker tracking, and the invention is applicable to existing solutions, so it is not required to use additional or specially designed software.

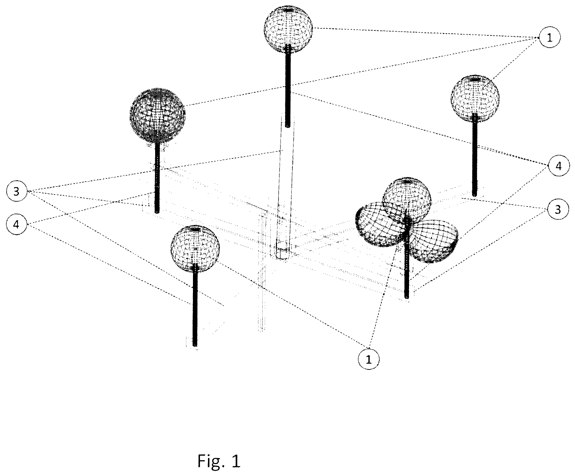

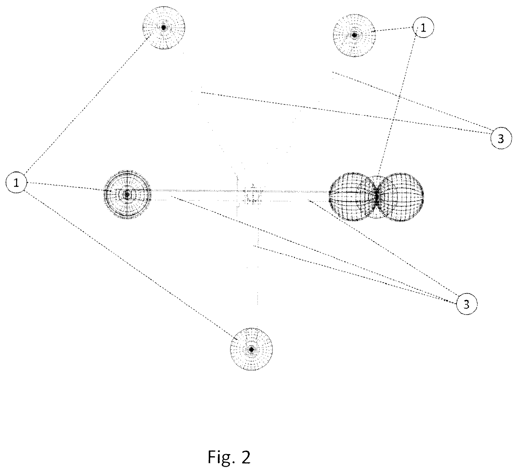

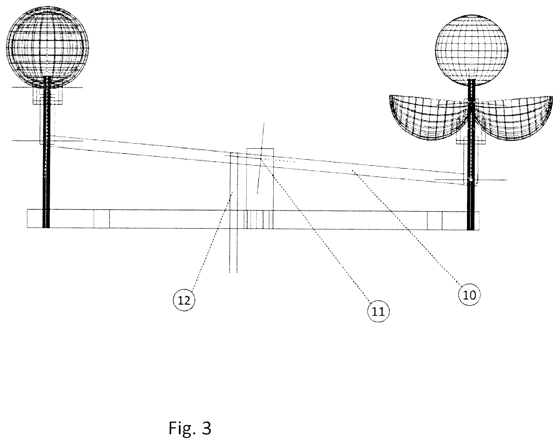

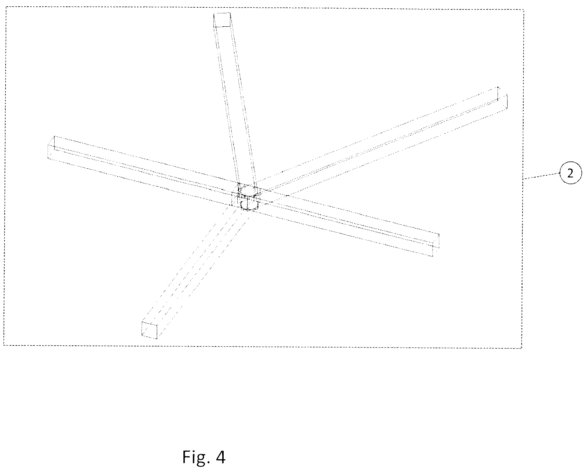

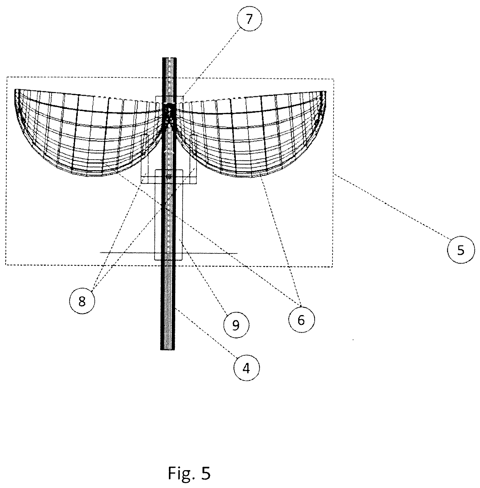

The solution is shown in the figures, where Fig. 1 shows the invention in a three-dimensional view with all spheres, Fig. 2 shows the invention in projection from top, Fig. 3 shows a combination of screening mechanisms, Fig. 4 shows a support, Fig. 5 shows the occluding mechanism in the open position.

The invention has been illustrated in exemplary set-up.

The manipulator is a device which performs modifications (manipulations) on a virtual object. A virtual object is any element of virtual reality which can be manipulated, in particular:
- the virtual scene and its parameters,
- objects (virtual elements) located in the scene and their features (such as color, texture, function),
- the perspective of a virtual person.

The term "manipulations" means making changes of the objects' location their features (color, texture, geometry, function). Function switching is understood as a modification of the type of function performed by the object (eg. switching from rotation change to scaling change) or changing the manipulated object to another one.

The term "multi-state" means the possibility of changing the marker's geometry or increase the number of types of geometry.

The invention may by applicable as:
- active (multi-state) manipulator in virtual reality systems based on optical tracking systems (eg. so called 3D Cave) - a three-dimensional equivalent of a computer mouse.
- extending of the user-virtual reality system interface with the possibility of active interaction - eg. capturing or modeling the objects;
- multi-state models in virtual reality, for example in medical applications - tracking the position of laparoscopic tools with the possibility of simulating the open / closed function (for scissors), or, the possibility of recording on / off status for diathermic tools.

The solution consists of five spheres [1] rigidly connected to the support [2] consisting of the arms [3]. The spheres [1] are mounted on pins [4], which connect to the arms [3]. The stick [4] is fitted with a occluding mechanism [5], which consists of two hemispheres [6] fixed to the sleeve's hinge [7]. The sleeve [7] is equipped with a pull-off spring that pulls it towards the sphere [1]. The opening of the hemisphere [6] is realized by the pull arms [8], which simultaneously deflect the hemisphere [6]. The pull arms [8] are attached to another sleeve [9] mounted on the stick [4].

The screening mechanisms [5] are connected by a connector [10] mounted on the hinge [11] so that the opening of one occluding mechanism [5] automatically closes the second mechanism [5], therefore only one screening mechanism [5] is open in a time and the other mechanism [5] is closed. The hinge [11] is equipped with a spring mechanism forcing one of the two settings of the screening mechanism [5] to be open and closed and prevents the occluding mechanism [5] from being in the intermediate position. The control is carried out by an additional handle [12].

Spheres [1] are covered with reflective material and the camera is equipped with an infrared emitter. The camera emits infrared radiation and is directed at the manipulator, which reflects the radiation back to the camera recording the image. The camera illuminates the marker, so it is visible. In the exemplary set-up the manipulator is mounted on the handle of a laparoscopic tool. In another example, the manipulator can be mounted to a glove to manipulate virtual reality.

## Claims

1. Multi-state marker/manipulator for optical systems tracking the location of the object **characterized in that** comprising:
At least five spheres [1] rigidly connected to the support [2] consisting of the arms [3], where two spheres are interchangeably covered and three are always visible and the spheres [1] are mounted on pins [4], which connect to the arms [3], two pins [4] are fitted with an occluding mechanism [5], which consists of two hemispheres [6] fixed to the sleeve's hinge [7], the sleeve [7] is equipped with a pull-off spring that pulls it towards the sphere [1]; the pull arms [8] perform the opening of the hemisphere [6], which simultaneously deflect the hemisphere [6], and the pull arms [8] are attached to another sleeve [9] mounted on the pin [4] and connected by the connector [10]

2. The invention of claim 1, is **characterized in that** the connector [10] has a hinge [11]

3. The invention of claim 1, is **characterized in that** it is equipped with additional handle [12]

4. The invention of claim 1, is **characterized in that** the optical tracker system is camera

5. The invention of claim 1, is **characterized in that** spheres [1] are covered with reflective material

6. The invention of claim 1, is **characterized in that** the arms [3] are rigidly connected
